# EUROPEAN PATENT APPLICATION

(11) **EP 3 466 922 A1**
(43) Date of publication of application: **10.04.2019**
(21) Application number: 17802736.3
(22) Date of filing: 22.05.2017
(51) Int. Cl.: C07C 231/02, B01J 27/10, B01J 27/12, B01J 31/02, C07C 235/06, C07C 235/08, C07C 235/16, C07C 235/46, C07C 259/06, C07C 269/06, C07C 271/22, C07D 209/40, C07D 213/40, C07D 215/40, C07D 295/185, C07D 307/54, C07D 317/66, C07D 333/24, C07B 61/00

(54) **CATALYST FOR CONVERTING ESTER TO AMIDE USING HYDROXYL GROUP AS ORIENTATION GROUP**

(30) Priority: 23.05.2016 JP 2016102354; 04.10.2016 JP 2016196193
(71) Applicant: Chubu University Educational Foundation, Kasugai-shi Aichi 487-8501 (JP)
(72) Inventor: YAMAMOTO, Hisashi, Kasugai-shi Aichi 487-8501 (JP); TSUJI, Hiroaki, Kasugai-shi Aichi 487-8501 (JP); KODAMA, Hidehiko, Higashiosaka-shi Osaka 577-0056 (JP); SUZUMA, Yoshinori, Higashiosaka-shi Osaka 577-0056 (JP)
(74) Representative: Lavoix
(86) International application number: PCT/JP2017/018991
(87) International publication number: WO 2017/204144

(57) **Abstract**

Provided is a method for amidating a hydroxy ester compound at a high chemical selectivity. The amidation reaction method for a hydroxy ester compound comprises, in the presence of a catalyst containing a compound of a transition metal of the group 4 or group 5 in the periodic table, reacting at least one kind of hydroxy ester compound selected from the group consisting of an α-hydroxy ester compound, a β-hydroxy ester compound, a γ-hydroxy ester compound and a δ-hydroxy ester compound with an amino compound so as to amidate an ester group having a hydroxyl group at the α-, β-, γ- or δ-position of the hydroxy ester compound.

## Description

### FIELD

The present invention relates to a method for the amidation of a hydroxy ester compound and a catalyst used for the amidation method.

### BACKGROUND

Metal-catalyzed reactions using a hydroxyl group as a directing group are one type of important reactions in organic synthetic chemistry, and superior synthetic reactions represented by asymmetric epoxidations and asymmetric hydrogenations have been realized (for example, refer to NPL 1). Such an effect of a hydroxyl group as a directing group is exhibited in not only epoxidations and hydrogenations, but also the functionalization of C-H bonds, epoxide opening reactions, cross-coupling reactions, and borations.

Conversion reactions of a carbonyl compound play a central role in organic synthesis. In these conversion reactions, it is known that the activation of a carbonyl group of a metal catalyst promotes a subsequent addition reaction, exchange reaction, or the like. However, an example in which the effect of a hydroxyl group as a directing group promoted the activation of a carbonyl group of a metal catalyst and a subsequent exchange reaction has not been known.

Amide groups are functional groups widely present in not only naturally-occurring products and pharmaceuticals, but also functional polymers, etc. Methods for the synthesis thereof have been intensively developed as an important research subject in synthetic chemistry. However, amide synthesis reactions still depend on the use of classical Schotten-Baumann reactions and stoichiometric amounts of peptide coupling reagents. Further, these methods have poor chemical selectivity, and are thus not suitable for the amidation of a molecule having two or more similar reactive sites. Under such a background, amidation reactions with high chemo selectivity represented by a catalyst method have been intensively developed, and a new future development in an amide synthesis strategy is desired.

### [CITATION LIST]

### [NON PATENT LITERATURE]

NPL 1: Hoveyda, A. H.; Evans, D. A.; Fu, G. C. Chem. Rev. 1993, 9, 1307.

### SUMMARY

### [TECHNICAL PROBLEM]

The primary object of the present invention is to provide a method for the amidation of a hydroxy ester compound with high chemoselectivity, and a catalyst used for the amidation method.

### [SOLUTION TO PROBLEM]

The present inventors have intensively studied to achieve the above object. As a result, the present inventors have found that an ester group having a hydroxyl group at the α-, β-, γ- or δ-position of the hydroxy ester compound is amidated with high chemoselectivity by allowing at least one hydroxy ester compound selected from the group consisting of α-hydroxy ester compounds, β-hydroxy ester compounds, γ-hydroxy ester compounds, and δ-hydroxy ester compounds to coexist with an amino compound in the presence of a catalyst comprising a compound of a transition metal of Group 4 or Group 5 of the periodic table. As a result of further research based on the above findings, the present invention has been accomplished.

In particular, the present invention provides the following embodiments:
Item 1. A method for the amidation of a hydroxy ester compound, comprising reacting at least one hydroxy ester compound selected from the group consisting of α-hydroxy ester compounds, β-hydroxy ester compounds, γ-hydroxy ester compounds, and δ-hydroxy ester compounds with an amino compound in the presence of a catalyst comprising a compound of a transition metal of Group 4 or Group 5 of the periodic table to amidate an ester group having a hydroxyl group at the α-, β-, γ- or δ-position of the hydroxy ester compound.
Item 2. The method for the amidation of a hydroxy ester compound according Item 1, wherein the hydroxy ester compound is represented by any of the following formulae (1a) to (1d): 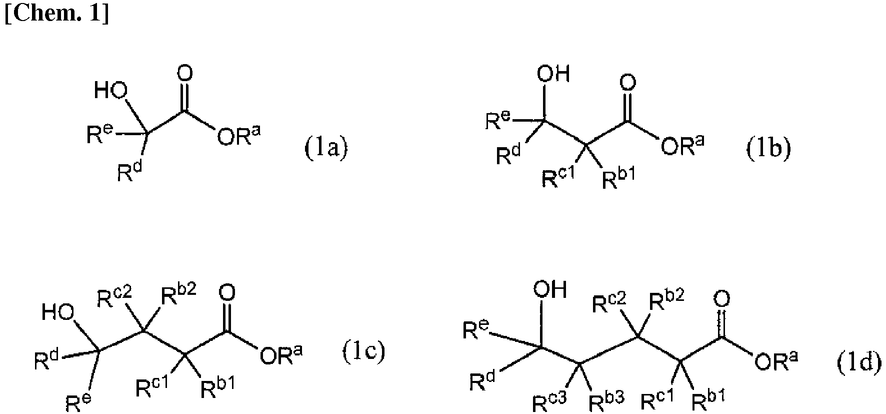 wherein group R^{a} represents an optionally substituted aliphatic group, an optionally substituted aromatic group, an optionally substituted alicyclic group, or an optionally substituted heterocyclic group; group R^{b1}, group R^{b2}, group R^{b3}, group R^{c1}, group R^{c2}, and group R^{c3} each independently represent a hydrogen atom, a halogen atom, a hydroxy group, an optionally substituted aliphatic group, an optionally substituted aromatic group, an optionally substituted alicyclic group, or an optionally substituted heterocyclic group; group R^{d} and group R^{e} each independently represent a hydrogen atom, an optionally substituted aliphatic group, an optionally substituted aromatic group, an optionally substituted alicyclic group, or an optionally substituted heterocyclic group; and group R^{b1} and group R^{c1}, group R^{b1} and group R^{b2}, group R^{b1} and group R^{b3}, group R^{b2} and group R^{c2}, group R^{b2} ;and group R^{b3}, group R^{b3} and group R^{c3}, group R^{b1} and group Rd, group R^{b2} and group R^{d}, or group R^{b3} and group R^{d} may be linked together to form a ring structure.
Item 3. The method for the amidation of a hydroxy ester compound according Item 1 or 2, wherein the amino compound is represented by the following formula (3): 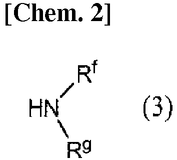 wherein group R^{f} and group R^{g} each independently represent a hydrogen atom, an optionally substituted aliphatic group, an optionally substituted aromatic group, an optionally substituted alicyclic group, an optionally substituted heterocyclic group, or group R^{f} and group R^{g} may form a saturated or unsaturated heterocycle together with a linked nitrogen atom, provided that the heterocycle may have a substituent.
Item 4. The method for the amidation of a hydroxy ester compound according any one of Items 1 to 3, wherein the compound of a transition metal is at least one selected from the group consisting of titanium compounds, zirconium compounds, hafnium compounds, vanadium compounds, niobium compounds, and tantalum compounds.
Item 5. The method for the amidation of a hydroxy ester compound according any one of Items 1 to 4, wherein the amount of the catalyst used is 100 mol% or less per 100 mol% of the hydroxy ester compound.
Item 6. The method for the amidation of a hydroxy ester compound according any one of Items 1 to 5, wherein amidation is performed in an organic solvent at a reaction temperature of 0 to 150 °C for a reaction time of 10 minutes to 72 hours.
Item 7. The method for the amidation of a hydroxy ester compound according any one of Items 1 to 6, wherein the hydroxy ester compound further comprises an ester group other than an α-hydroxy ester, a β-hydroxy ester, a γ-hydroxy ester, or a δ-hydroxy ester.
Item 8. The method for the amidation of a hydroxy ester compound according any one of Items 1 to 7, wherein the amino compound is an amino acid, a salt thereof, an amino-acid ester, or a salt thereof.
Item 9. The method for the amidation of a hydroxy ester compound according any one of Items 1 to 8, wherein the hydroxy ester compound further comprises an amino group.
Item 10. The method for the amidation of a hydroxy ester compound according any one of Items 1 to 9, wherein at least either one of the hydroxy ester compound and the amino compound is an oligopeptide comprising 2 or more amino acid residues.
Item 11. The method for the amidation of a hydroxy ester compound according any one of Items 1 to 10, wherein an amide compound obtained by amidation is at least one selected from the group consisting of the following general formula (4a) to (4d) 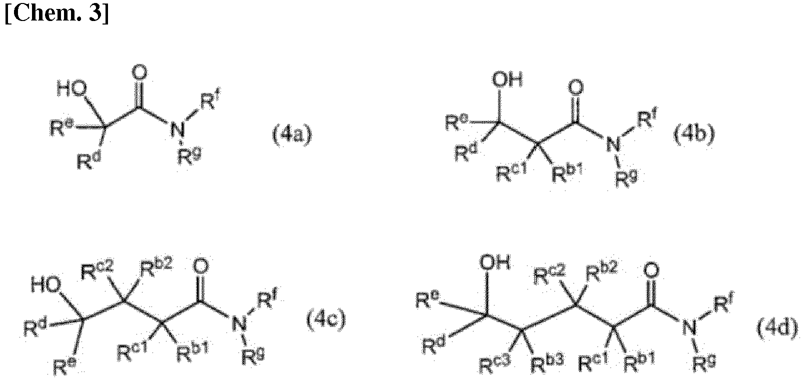 wherein group R^{a} represents an optionally substituted aliphatic group, an optionally substituted aromatic group, an optionally substituted alicyclic group, or an optionally substituted heterocyclic group; group R^{b1}, group R^{b2}, group R^{b3}, group R^{c1}, group R^{c2}, and group R^{c3} each independently represent a hydrogen atom, a halogen atom, a hydroxy group, an optionally substituted aliphatic group, an optionally substituted aromatic group, an optionally substituted alicyclic group, or an optionally substituted heterocyclic group; group R^{d} and group R^{e} each independently represent a hydrogen atom, an optionally substituted aliphatic group, an optionally substituted aromatic group, an optionally substituted alicyclic group, or an optionally substituted heterocyclic group; group R^{b1} and group R^{c1}, group R^{b1} and group R^{b2}, group R^{b1} and group R^{b3}, group R^{b2} and group R^{c2}, group R^{b2} and group R^{b3}, group R^{b3} and group R^{c3}, group R^{b1} and group R^{d}, group R^{b2} and group R^{d}, or group R^{b3} and group R^{d} may be linked together to form a ring structure; and group R¹ and group R^{g} each independently represent a hydrogen atom, an optionally substituted aliphatic group, an optionally substituted aromatic group, an optionally substituted alicyclic group, an optionally substituted heterocyclic group, or group R^{f} and group R^{g} may form a saturated or unsaturated heterocycle together with a linked nitrogen atom, provided that the heterocycle may have a substituent.
Item 12. A catalyst comprising a compound of a transition metal of Group 4 or Group 5 of the periodic table, the catalyst being used in a method for the amidation of a hydroxy ester compound, and the method comprising reacting at least one hydroxy ester compound selected from the group consisting of an α-hydroxy ester compound, a β-hydroxy ester compound, a γ-hydroxy ester compound, and a δ-hydroxy ester compound with an amino compound to amidate an ester group having a hydroxyl group at the α-, β-, γ- or δ-position of the hydroxy ester compound.

### ADVANTAGEOUS EFFECTS OF INVENTION

According to the present invention, provided are a method for the amidation of a hydroxy ester compound with high chemoselectivity, and a catalyst capable of suitably promoting the amidation.

### DESCRIPTION OF EMBODIMENTS

The method for the amidation of a hydroxy ester compound of the present invention comprises reacting at least one hydroxy ester compound selected from the group consisting of α-hydroxy ester compounds, β-hydroxy ester compounds, γ-hydroxy ester compounds, and δ-hydroxy ester compounds with an amino compound in the presence of a catalyst comprising a compound of a transition metal of Group 4 or Group 5 of the periodic table to amidate an ester group having a hydroxyl group at the α-, β-, γ- or δ-position of the hydroxy ester compound. In particular, for example, an α-hydroxy ester compound and an amino compound are reacted to amidate an ester group having a hydroxyl group at the α-position of the hydroxy ester compound. Likewise, a β-hydroxy ester compound and an amino compound are reacted to amidate an ester group having a hydroxyl group at the β-position of the hydroxy ester compound. A γ-hydroxy ester compound and an amino compound are reacted to amidate an ester group having a hydroxyl group at the γ-position of the hydroxy ester compound. A δ-hydroxy ester compound and an amino compound are reacted to amidate an ester group having a hydroxyl group at the δ-position of the hydroxy ester compound.

In the method for the amidation of a hydroxy ester compound of the present invention, the hydroxy ester compound is not particularly limited so long as the hydroxy ester compound has a hydroxyl group at at least one of the α-, β-, γ- and δ-positions. Further, the amino compound is not particularly limited so long as the amino compound can react with the ester group to form an amide group, but is preferably, for example, a primary amine or a secondary amine.

When the method for the amidation of a hydroxy ester compound of the present invention is specifically represented by general formulae, the method comprises reacting a hydroxy ester compound represented by any of the following formulae (1a) to (1d) (hereinafter, referred to sometimes as α-hydroxy ester compound (1a), β-hydroxy ester compound (1b), γ-hydroxy ester compound (1c), and δ-hydroxy ester compound (1d), respectively): 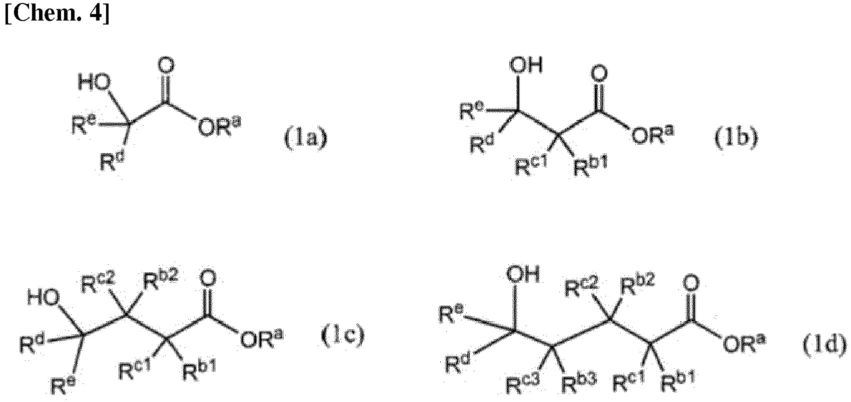 with
an amino compound of the following general formula (3) (hereinafter, referred to sometimes as amino compound (3)): 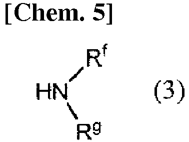 in the presence of a catalyst comprising a compound of a transition metal of Group 4 or Group 5 of the periodic table to produce
a hydroxy amide compound represented by any of the following formulae (4a) to (4d) (hereinafter, referred to sometimes as α-hydroxy amide compound (4a), β-hydroxy amide compound (4b), γ-hydroxy amide compound (4c), δ-hydroxy amide compound (4d)): 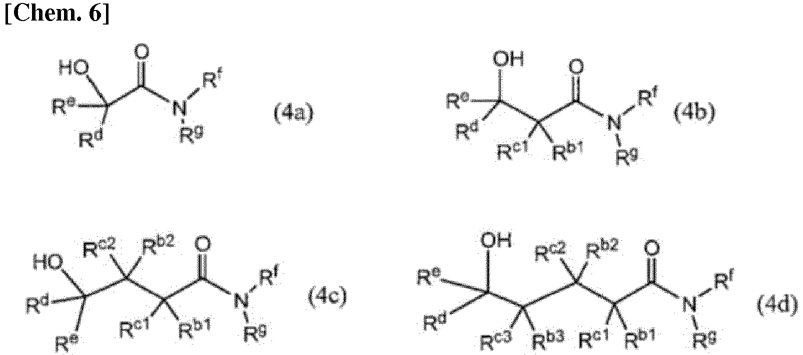 Referring to the method for the amidation of a β-hydroxy ester compound as an example, as shown in the following reaction formula (A), a β-hydroxy ester compound (1b) and an amino compound (3) are reacted in the presence of a catalyst comprising a compound of a transition metal of Group 4 or Group 5 of the periodic table to suitably produce a β-hydroxy amide compound (4b).

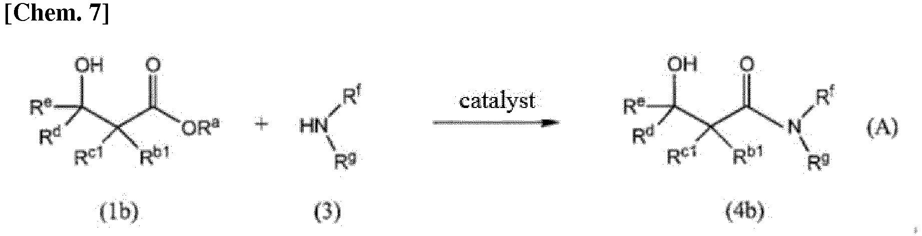

Though a general formula is omitted, via a similar reaction, the hydroxy ester compound (1a), the hydroxy ester compound (1c), or the hydroxy ester compound (1d) reacts with the amino compound (3) in the presence of a catalyst comprising a compound of a transition metal of Group 4 or Group 5 of the periodic table to suitably produce the α-hydroxy amide compound (4a), γ-hydroxy amide compound (4c), or the δ-hydroxy amide compound (4d).

The term "to" as used herein represents a numerical value range from not less than the value shown on the left side of "to" to not more than the value shown on the right side of "to". For example, the numerical value range "X to Y" refers to the range from not less than X to not more than Y.

According to the amidation method of the present invention, it is possible to amidate hydroxy ester compounds with high chemoselectivity, presumably because, referring to the reaction of the β-hydroxy ester compound (1b) as an example, as shown in, for example, the following reaction formula (B), a catalyst [M] coordinates to the hydroxyl group at the β-position of the β-hydroxy ester compound (1b) and the oxygen atom of a carbonyl group (general formula (1b')), whereby the amino compound (3) highly selectively reacts with the carbonyl carbon of the β-hydroxy ester compound (1b) and the amidation effectively proceeds in the amidation method of the present invention.

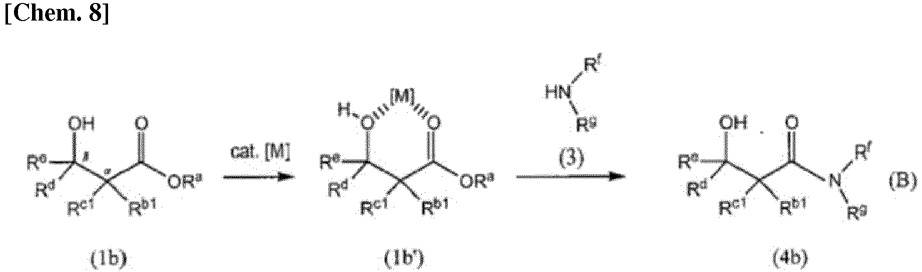

Though a general formula is omitted, like the reaction of the β-hydroxy ester compound (1b), the amino compound (3) highly selectively reacts with the carbonyl carbon of the α-hydroxy ester compound (1a) and the amidation effectively proceeds, presumably because the catalyst [M] coordinates to the hydroxyl group at the α-position of the α-hydroxy ester compound (1a) and the oxygen atom of a carbonyl group. Likewise, the amino compound (3) highly selectively reacts with the carbonyl carbon of the γ-hydroxy ester compound (1c) and the amidation effectively proceeds, presumably because the catalyst [M] coordinates to the hydroxyl group at the γ-position of the γ-hydroxy ester compound (1c) and the oxygen atom of a carbonyl group. Likewise, the amino compound (3) highly selectively reacts with the carbonyl carbon of the δ-hydroxy ester compound (1d) and the amidation effectively proceeds, presumably because the catalyst [M] coordinates to the hydroxyl group at the δ-position of the δ-hydroxy ester compound (1d) and the oxygen atom of a carbonyl group.

Each group R^{a} of the hydroxy ester compounds (1a) to (1d) represents an optionally substituted aliphatic group, an optionally substituted aromatic group, an optionally substituted alicyclic group, or an optionally substituted heterocyclic group. Further, group R^{b1}, group R^{b2}, group R^{b3}, group R^{c1}, group R^{c2}, and group R^{c3} each independently represent a hydrogen atom, a halogen atom, a hydroxyl group, an optionally substituted aliphatic group, an optionally substituted aromatic group, an optionally substituted alicyclic group, or an optionally substituted heterocyclic group. Group R^{d} and group R^{e} each independently represent a hydrogen atom, an optionally substituted aliphatic group, an optionally substituted aromatic group, an optionally substituted alicyclic group, or an optionally substituted heterocyclic group. Group R^{b1} and group R^{c1}, group R^{b1} and group R^{b2}, group R^{b1} and group R^{b3}, group R^{b2} and group R^{c2}, group R^{b2} and group R^{b3}, group R^{b3} and group R^{c3}, group R^{b1} and group R^{d}, group R^{b2} and group R^{d}, or group R^{b3} and group R^{d} each may be linked together to form a ring structure (e.g., an alicyclic structure, a heterocyclic structure, an aromatic ring structure). The number of carbon atoms forming the ring structure is preferably 5 to 10. Specific examples of the ring structure include a benzene ring.

The substituents which group R^{a}, group R^{b1}, group R^{b2}, group R^{b3}, group R^{c1}, group R^{c2}, group R^{c3}, group R^{d}, and group R^{e} may have (the substituents of an aliphatic group, an alicyclic group, and a heterocyclic group) are not particularly limited so long the amidation of the present invention can proceed, and each independently represent, for example, an alkyl group (e.g., a linear or branched alkyl group having 1 to 10 carbon atoms), such as a methyl group, an ethyl group, a propyl group, an isopropyl group, or a butyl group; an alkenyl group (e.g., a linear or branched alkenyl group having 1 to 10 carbon atoms), such as an allyl group, a propenyl group, an isopropenyl group, a 1-butenyl group, a 2-butenyl group, an alkynyl group (e.g., a linear or branched alkynyl group having 1 to 10 carbon atoms, such as a propargyl group), an alkoxy group (e.g., a linear or branched alkoxy group having 1 to 10 carbon atoms, such as a methoxy group, an ethoxy group, a propoxy group, a butoxy group, a sec-butoxy group, or a tert-butoxy group), an amino group, a hydroxyl group, a halogen atom, a nitro group, a thiol group, a cyano group, or a phenyl group. Further, when the aliphatic group, the aromatic group, the alicyclic group, or the heterocyclic group of group R^{a}, group R^{b1}, group R^{b2}, group R^{b3}, group R^{c1}, group R^{c2}, group R^{c3}, group R^{d}, and group R^{e} has substituents, the number of the substituents is not particularly limited. These groups may each independently have, for example, 1 to 10, 1 to 5, 1 to 3, 1 or 2, or 1 substituent. Further, when each group has a plurality of substituents, the substituents may consist of one type or two or more types thereof. The aliphatic group and the aromatic group each may contain a hetero atom. Further, the aliphatic group, the alicyclic group, and the heterocyclic group may be saturated or unsaturated.

In the present invention, when the hydroxy ester compound has an amino group (e.g., when the hydroxy ester compound has an amino acid or a derivative thereof (esterified amino acid or the like)), an amide compound obtained by the amidation method of the present invention and a hydroxy ester compound can be subjected to an amidation reaction. In other words, in the present invention, a hydroxy ester compound having an amino group is used. Thus, the resulting amide compound can be used as an amino compound, and further, the reaction thereof with the hydroxy ester compound can be repeated. In the present invention, various structures of hydroxy ester compounds to be added by repetition can be selected to carry out the amidation reactions, whereby amino compounds comprising amino acid units having desired structures linked by peptide bonds can be synthesized and thus a desired oligopeptide can be produced with high chemo selectivity.

In the hydroxy ester compound having an amino group, the amino group is preferably protected by a protecting group. When amidation is repeated, the deprotection of this protecting group can be carried out as necessary after an amide compound is formed by the amidation method and by the time when the amide compound is reacted as the amino compound with a hydroxy ester compound.

Typical examples of the protecting group of the amino group include acyl groups, carbamates, amides, aryl groups, aralkyl groups, and alkenyl groups, which may be substituted or non-substituted. The names of the protecting groups include the names of groups linked to the N atom of an amino group and the names of groups including an N atom. Both types of names are included in the following names.

Specific groups of the acyl groups include a benzoyl group (Bz), an ortho-methoxybenzoyl group, a 2,6-dimethoxybenzoyl group, a para-methoxybenzoyl group (PMPCO), a 2,2,2-trichloroethoxycarbonyl group (Troc), a phthaloyl group (Phth), and a 9-fluorenylmethyloxycarbonyl group (Fmoc). Specific groups of the carbamates include a tert-butoxycarbonyl group (Boc), a benzyloxycarbonyl group (Cbz), methylcarbamate, ethylcarbamate, 2-trimethylsilylethylcarbamate, 2-phenylethylcarbamate, 1-(1-adamantyl)-1-methylethylcarbamate, 1-(3,5-di-t-butylphenyl)-1-methylethylcarbamate, vinylcarbamate, allylcarbamate, N-hydroxypiperidinylcarbamate, p-methoxybenzylcarbamate, p-nitrobenzylcarbamate, [2-(1,3-dithianyl)methylcarbamate, m-nitrophenylcarbamate, 3,5-dimethoxybenzylcarbamate, and o-nitrobenzylcarbamate. Specific groups of the amides include aceto amide, o-(benzoyloxymethyl)benzamide, 2-[(t-butyldiphenylsiloxy)methyl]benzamide, 2-toluenesulfonamide, 4-toluenesulfonamide, 2-nitrobenzenesulfonamide, 4-nitrobenzenesulfonamide, tert-butylsulfinylamide, 4-toluenesulfonamide, 2-(trimethylsilyl)ethanesulfonamide, benzylsulfonamide, aromatic or heterocyclic carboxylic acids, and acyl groups derived from sulfonic acid. Specific groups of the aryl groups include a 2,4-dinitrophenyl group (2,4-DNP). Specific groups of the aralkyl group include a benzyl group (Bn) and a phenethyl group. Specific groups of the alkenyl groups include a vinyl group, an allyl group, and a hexenyl group.

Further, from the viewpoint of the deprotection means, examples of the protecting group include protecting groups which can be deprotected by at least one of deprotection by hydrogenation, deprotection by a weak acid, deprotection by fluorine ions, deprotection by a one-electron oxidant, deprotection by hydrazine, and deprotection by oxygen. Examples of the deprotection by hydrogenation include (a) a method of deprotection by reduction using a metal catalyst, such as palladium, palladium-carbon, palladium hydroxide, or palladium hydroxide-carbon, as a reduction catalyst in the presence of a hydrogen gas, and (b) a method of deprotection by reduction using a hydrogenation reducing agent, such as sodium borohydride, lithium aluminum hydride, lithium borohydride, or diborane in the presence of a metal catalyst, such as palladium, palladium-carbon, palladium hydroxide, or palladium hydroxide-carbon.

Preferable specific examples of the protecting group include a tert-butoxycarbonyl group (Boc), a benzyl group (Bn), a benzyloxycarbonyl group (Cbz), a benzoyl group (Bz), a 2,2,2-trichloroethoxycarbonyl group (Troc), a 2,4-dinitrophenyl group (2,4-DNP), a phthaloyl group (Phth), a para-methoxybenzoyl group (PMPCO), and a 9-fluorenylmethyloxycarbonyl group (Fmoc).

Group R^{a} is preferably an optionally substituted aliphatic group having 1 to 20 carbon atoms, an optionally substituted aromatic group having 4 to 20 carbon atoms, an optionally substituted alicyclic group having 3 to 20 carbon atoms, or an optionally substituted heterocyclic group having 2 to 20 carbon atoms, and more preferably an optionally substituted aliphatic group having 1 to 10 carbon atoms, an optionally substituted aromatic group having 4 to 10 carbon atoms, an optionally substituted alicyclic group having 3 to 10 carbon atoms, or an optionally substituted heterocyclic group having 2 to 10 carbon atoms. The substituents of group R^{a} are the same as stated above. Specific examples of group R^{a} include a linear or branched alkyl group having 1 to 10 carbon atoms, such as a methyl group, an ethyl group, a propyl group, an isopropyl group, a butyl group, a sec-butyl group, or a tert-butyl group, a phenylalkyl group such as a phenyl group or a benzyl group comprising an alkyl portion consisting of a linear or branched alkyl group having 1 to 10 carbon atoms, a linear or branched chain alkenyl group having 1 to 10 carbon atoms, such as an allyl group, and a linear or branched chain alkynyl group having 1 to 10 carbon atoms, such as an propargyl. The substituents of group R^{a} are the same as stated above.

Group R^{b1}, group R^{b2}, group R^{b3}, group R^{c1}, group R^{c2}, and group R^{c3} each independently represent preferably a hydrogen atom, a halogen atom, a hydroxyl group, an optionally substituted aliphatic group having 1 to 20 carbon atoms, an optionally substituted aromatic group having 4 to 20 carbon atoms, an optionally substituted alicyclic group having 3 to 20 carbon atoms, or an optionally substituted heterocyclic group having 2 to 20 carbon atoms, and more preferably a hydrogen atom, a halogen atom, a hydroxyl group, an optionally substituted aliphatic group having 1 to 10 carbon atoms, an optionally substituted aromatic group having 4 to 10 carbon atoms, an optionally substituted alicyclic group having 3 to 10 carbon atoms, or an optionally substituted heterocyclic group having 2 to 10 carbon atoms. As specific examples, group R^{b1}, group R^{b2}, group R^{b3}, group R^{c1}, group R^{c2}, and group R^{c3} each independently represent a hydrogen atom, a hydroxyl group, a nitro group, a thiol group, a cyano group, a phenyl group; a halogen atom such as a fluorine atom, a chlorine atom, a bromine atom, or an iodine atom; a linear or branched alkyl group having 1 to 10 carbon atoms, such as a methyl group, an ethyl group, a propyl group, an isopropyl group, or a butyl group; a linear or branched alkenyl group having 1 to 10 carbon atoms, such as an ethylene group, a propylene group, or a butylene group; an alkynyl group having 1 to 10 carbon atoms, such as a propargyl group; or a linear or branched alkoxy group having 1 to 10 carbon atoms, such as a methoxy group, an ethoxy group, a propoxy group, a butoxy group, a sec-butoxy group, or a tert-butoxy group. The substituents of group R^{b1}, group R^{b2}, group R^{b3}, group R^{c1}, group R^{c2}, and group R^{c3} are the same as stated above.

Group R^{d} and group R^{e} each independently represent preferably a hydrogen atom, an optionally substituted aliphatic group having 1 to 20 carbon atoms, an optionally substituted aromatic group having 4 to 20 carbon atoms, an optionally substituted alicyclic group having 3 to 20 carbon atoms, or an optionally substituted heterocyclic group having 2 to 20 carbon atoms, and more preferably a hydrogen atom, an optionally substituted aliphatic group having 1 to 10 carbon atoms, an optionally substituted aromatic group having 4 to 10 carbon atoms, an optionally substituted alicyclic group having 3 to 10 carbon atoms, or an optionally substituted heterocyclic group having 2 to 10 carbon atoms. As specific examples thereof, group R^{d} and group R^{e} each independently represent a hydrogen atom, a hydroxyl group, a nitro group, a thiol group, a cyano group, a phenyl group; a halogen atom, such as a fluorine atom, a chlorine atom, a bromine atom, or an iodine atom; a linear or branched alkyl group having 1 to 10 carbon atoms, such as a methyl group, an ethyl group, a propyl group, an isopropyl group, or a butyl group; a linear or branched alkenyl group having 1 to 10 carbon atoms such as an ethylene group, a propylene group, or a butylene group; a linear or branched alkynyl group having 1 to 10 carbon atoms, such as a propargyl group; or a linear or branched alkoxy group having 1 to 10 carbon atoms, such as a methoxy group, an ethoxy group, a propoxy group, a butoxy group, a sec-butoxy group, or a tert-butoxy group. The substituents of group R^{d} and group R^{e} are the same as stated above.

Group R^{f} and group R^{g} of amino compound (3) each independently represent a hydrogen atom, an optionally substituted aliphatic group, an optionally substituted aromatic group, an optionally substituted alicyclic group, or an optionally substituted heterocyclic group. Further, group R^{f} and group R^{g} may form a saturated or unsaturated heterocycle together with a linked nitrogen atom. The heterocyclic group may have a substituent.

The substituents of the heterocycle formed by group R^{f} and group R^{g} together with a linked nitrogen atom are not particularly limited so long as an amide group can be formed by a reaction with a hydroxy ester compound. The substituents each independently represent, for example, an alkyl group (e.g., a linear or branched alkyl group having 1 to 10 carbon atoms), an alkenyl group (e.g., a linear or branched alkenyl group having 1 to 10 carbon atoms), an alkynyl group (e.g., a linear or branched alkynyl group having 1 to 10 carbon atoms), an alkoxy group (e.g., a linear or branched alkoxy group having 1 to 10 carbon atoms), a hydroxyl group, a halogen atom, a nitro group, a thiol group, a cyano group, a linear or branched alkylthio group having 1 to 10 carbon atoms, an optionally substituted amino group, an optionally substituted amide group, an optionally substituted guadinino group, group-COOR^{a} (R^{a} is the same as above), an optionally substituted aryl group, or an optionally substituted heterocyclic group. The definitions of the substituents of an optionally substituted amino group, an optionally substituted amide group, an optionally substituted guadinino group, an optionally substituted aryl group, and an optionally substituted heterocyclic group are the same as those of group R^{f} and group R^{g}. The aryl group may be a phenyl group. The heterocyclic group may be an indolyl group or an imidazolyl group. Further, when the aliphatic group, the aromatic group, the alicyclic group, or the heterocyclic group of the heterocycle formed by group R^{f} and group R^{g} together with a linked nitrogen atom has a substituent, the number of the substituents is not particularly limited. These groups may each independently have, for example, 1 to 10, 1 to 5, 1 to 3, 1 or 2, or 1 substituent. Further, when each group has a plurality of substituents, the substituents may consist of one type or two or more types thereof. The aliphatic group and the aromatic group each may contain a hetero atom. Further, the aliphatic group, the alicyclic group, and the heterocyclic group may be saturated or unsaturated.

Group R^{f} and group R^{g} of amino compound (3) each independently represent preferably a hydrogen atom, an optionally substituted aliphatic group having 1 to 20 carbon atoms, an optionally substituted aromatic group having 4 to 20 carbon atoms, an optionally substituted alicyclic group having 3 to 20 carbon atoms, or an optionally substituted heterocyclic group having 2 to 20 carbon atoms, and more preferably a hydrogen atom, an optionally substituted aliphatic group having 1 to 10 carbon atoms, an optionally substituted aromatic group having 4 to 10 carbon atoms, an optionally substituted alicyclic group having 3 to 10 carbon atoms, or an optionally substituted heterocyclic group having 2 to 10 carbon atoms. However, it is not preferable that group R^{f} and group R^{g} each be a hydrogen atom (i.e., when amino compound (3) is ammonia) due to the low boiling point thereof. The substituents of group R^{f} and group R^{g} are the same as stated above.

The aliphatic group, the aromatic group, the alicyclic group, and the heterocyclic group of group R^{a} to group R^{g} of each of the above hydroxy ester compounds (1a) to (1d) and amino compound (3) are not particularly limited unless the progress of amidation is inhibited thereby (i.e., none of amide compound (4a) to (4d) can be substantially obtained). Specific examples of the aliphatic group include an alkyl group (e.g., a linear or branched alkyl group having 1 to 20 carbon atoms), an alkenyl group (e.g., a linear or branched alkenyl group having 1 to 20 carbon atoms), an alkynyl group (e.g., a linear or branched alkynyl group having 1 to 20 carbon atoms), am alkoxy group (e.g., a linear or branched alkoxy group having 1 to 20 carbon atoms), and a group:-SR (R is, for example, a linear or branched alkyl group having about 1 to 10 carbon atoms). Specific examples of the aromatic group include a phenyl group, a naphthyl group, an imidazole group, a pyrazole group, an oxazole group, a thiazole group, an imidazoline group, a pyradine group, an indole group, a pyrrole group, and a pyridyl group. Specific examples of the alicyclic group include cycloalkyl group having 3 to 8 carbon atoms, such as a cyclopropyl group, a cyclobutyl group, a cyclopentyl group, or a cyclohexyl group; a bicyclic alkenyl group, such as a norbornyl group or a norbomadienyl group, or a furan group. Specific examples of the heterocyclic group include an aziridine group, an oxirane group, an oxolane group, an oxole group, a thiol group (thiophenyl group), or a tetrahydrofuryl group. As stated above, these groups may have the above substituents. Further, the aliphatic group, the aromatic group, alicyclic group, and the heterocyclic group each may include various bonds, such as an ester bond, an amide bond, and an ether bond.

Specific examples of the saturated or unsaturated heterocycle formed by group R^{f} and group R^{g} together with a linked nitrogen atom include pyrrolinyl, pyrrolyl, 2,3-dihydro-1H-pyrrolyl, piperidinyl, piperadinyl, homopiperadinyl, morpholino, thiomorpholino, 1,2,4,6-tetrahydropyridyl, hexahydropyrimidyl, hexahydropyridazyl, 1,2,4,6-tetrahydropyridyl, 1,2,4,6-tetrahydropyridazyl, 3,4-dihydropyridyl, imidazolyl, 4,5-dihydro-1H-imidazolyl, 2,3-dihydro-1H-imidazolyl, pyrazolyl, 4,5-dihydro-1H-pyrazolyl, 2,3-dihydro-1H-pyrazolyl, oxazolyl, 4,5-dihydro-1,3-oxazolyl, 2,3-dihydro-1,3-oxazolyl, 2,5-dihydro-1,3-oxazolyl, thiazolyl, 4,5-dihydro-1,3-thiazolyl, 2,3-dihydro-1,3-thiazolyl, 2,5-dihydro-1,3-thiazolyl, and other 5- or 6-membered saturated or unsaturated heterocyclic groups.

In the present invention, the amino compound is particularly preferably an amino acid or a salt thereof, or an amino acid ester or a salt thereof. According to the method for producing an amide compound of the present invention, the amide compound can be produced with high chemo selectivity, and thus, a peptide can be synthesized with high chemo selectivity by reacting a hydroxy ester compound with an amino acid or a salt thereof, or an amino acid ester or a salt thereof having an asymmetric center. The above amino compound (3) encompasses an amino acid or a salt thereof, or an amino acid ester or a salt thereof.

The amino acid is not particularly limited, and may be a publicly known amino acid, such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, or an amino acid oligomer (typically, from a dimer to a decamer) comprising at least one thereof. Further, examples of the amino acid ester include esters obtained by the esterification of the carboxyl group of the above amino acid by a linear or branched alkyl group having 1 to 10 carbon atoms, a linear or branched alkynyl group having 1 to 10 carbon atoms such as a propargyl group, or an aryl group. Furthermore, the salt of an amino acid or the salt of an amino acid ester each may be a hydrochloride, a sulfate, an oxalate, or a phosphate of the above amino acid or amino acid ester.

The molar ratio of the hydroxy ester compound and the amino compound in the method of amidation of the present invention is not particularly limited. The amino compound may be used in the amount of 1 to 10 mol, preferably 1 to 5 mol, with respect to 1 mol of the hydroxy ester compound.

When an amino group derived from a hydroxy ester compound is used to produce an amino compound from an amide compound after amidation, and the amino compound reacts with the above hydroxy ester compound to produce a dipeptide, or when a plurality of peptide bonds are formed by repeating these procedures to produce an oligopeptide, the excessive use of the hydroxy ester compound with respect to the amino compound used for the reactions is advantageous in cost. In other words, in the present invention, a hydroxy ester compound having an amino group can be used as an amino acid unit to be sequentially bonded to the amino compound, whereby the hydroxy ester compound having an amino group derived from the amino acid can be prepared relatively inexpensively.

The catalyst used in the amidation method of the present invention is not particularly limited so long as the catalyst comprises a compound of a transition metal of Group 4 or Group 5 of the periodic table (i.e., a transition metal compound comprising a metal element of Group 4 or Group 5 of the periodic table). Examples of the transition metal compound include titanium compounds, zirconium compounds, hafnium compounds, vanadium compounds, niobium compounds, and tantalum compounds. The catalyst may comprise one or two or more of these transition metal compounds.

Thereamong, at least one of tantalum compounds, niobium compounds, vanadium compounds, and titanium compounds is preferably contained in the catalyst from the viewpoint of increasing the yield of a hydroxy amide compound while maintaining excellent chemoselectivity of the amidation method of the present invention. At least one of tantalum compounds and niobium compounds is more preferably contained therein.

The tantalum compound contained in the catalyst is not particularly limited so long as the amidation of the hydroxy ester compound proceeds. Specific examples of the tantalum compounds include TaX¹₅ (wherein, five X¹s each independently represent an alkoxy group, a halogen atom, an allyloxy group, a group:-SR, a group:-NRR', or the like, and generally represent the same group). The alkoxy group of X¹ is preferably a linear or branched alkoxy group having 1 to 10 carbon atoms, more preferably a linear or branched alkoxy group having 1 to 5 carbon atoms, even more preferably a linear or branched alkoxy group having 1 to 3 carbon atoms. Further, the allyloxy group is preferably a linear or branched allyloxy group having 1 to 20 carbon atoms, more preferably a linear or branched allyloxy group having 1 to 15 carbon atoms, even more preferably an allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent a hydrogen atom, or a linear or branched chain alkyl group, alkenyl group, or aryl group having about 1 to 10 carbon atoms. Thereamong, tantalum alkoxide compounds (e.g., X¹ is an alkoxy group) are preferable. The tantalum compounds may be used alone or in combination of two or more.

The niobium compounds are not particularly limited so long as the amidation of the hydroxy ester compound proceeds. Specific examples of the niobium compounds include NbX²₅ (wherein five X²s each independently represent an alkoxy group, a halogen atom, an allyloxy group, a group:-SR, a group:-NRR', or the like and, and generally represent the same group). The alkoxy group of X² is preferably a linear or branched alkoxy group having 1 to 10 carbon atoms, more preferably a linear or branched alkoxy group having 1 to 5 carbon atoms, even more preferably a linear or branched alkoxy group having 1 to 3 carbon atoms. The allyloxy group is preferably a linear or branched allyloxy group having 1 to 20 carbon atoms, more preferably a linear or branched allyloxy group having 1 to 15 carbon atoms, even more preferably a linear or branched allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent a hydrogen atom, or a linear or branched chain alkyl group, alkenyl group, or aryl group having about 1 to 10 carbon atoms. Thereamong, niobium alkoxide compounds (e.g., X² is an alkoxy group) are preferable. The niobium compounds may be used alone or in combination of two or more.

The vanadium compounds are not particularly limited so long as the amidation of the hydroxy ester compound proceeds. Specific examples of the vanadium compounds include VOX³₃ (wherein three X³ each independently represent an alkoxy group, a halogen atom, an allyloxy group, a group:-SR, a group:-NRR', or the like, and generally represent the same group). The alkoxy group of X³ is preferably a linear or branched alkoxy group having 1 to 10 carbon atoms, more preferably a linear or branched alkoxy group having 1 to 5 carbon atoms, even more preferably a linear or branched alkoxy group having 1 to 3 carbon atoms. The allyloxy group is preferably a linear or branched allyloxy group having 1 to 20 carbon atoms, more preferably a linear or branched allyloxy group having 1 to 15 carbon atoms, even more preferably a linear or branched allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent a hydrogen atom, or a linear or branched chain alkyl group, alkenyl group, or aryl group having about 1 to 10 carbon atoms. Thereamong, vanadium alkoxide compounds (e.g., X³ is an alkoxy group) are preferable. The vanadium compounds may be used alone or in combination of two or more.

The titanium compounds are not particularly limited so long as the amidation of the hydroxy ester compound proceeds. Specific examples of the titanium compounds include TiX⁴₄ (wherein four X⁴s each independently represent an alkoxy group, a halogen atom, an allyloxy group, a group:-SR, a group:-NRR', or the like, and generally represent the same group). The alkoxy group of X⁴ is preferably a linear or branched alkoxy group having 1 to 10 carbon atoms, more preferably a linear or branched alkoxy group having 1 to 5 carbon atoms, even more preferably a linear or branched alkoxy group having 1 to 3 carbon atoms. The allyloxy group is preferably a linear or branched allyloxy group having 1 to 20 carbon atoms, more preferably a linear or branched allyloxy group having 1 to 15 carbon atoms, even more preferably a linear or branched allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent, a hydrogen atom, a linear or branched chain alkyl group, alkenyl group, or aryl group having about 1 to 10 carbon atoms. Thereamong, titanium alkoxide compounds (e.g., X⁴ is an alkoxy group) are preferable. The titanium compounds may be used alone or in combination of two or more.

The zirconium compounds are not particularly limited so long as the amidation of the hydroxy ester compound proceeds. Specific examples of the zirconium compound include ZrX⁵₄ (wherein four X⁵s each independently represent an alkoxy group, a halogen atom, an allyloxy group, a group:-SR, a group:-NRR' or the like, and generally represent the same group). The alkoxy group of X⁵ is preferably a linear or branched alkoxy group having 1 to 10 carbon atoms, more preferably a linear or branched alkoxy group having 1 to 5 carbon atoms, even more preferably a linear or branched alkoxy group having 1 to 4 carbon atoms. The allyloxy group is preferably a linear or branched allyloxy group having 1 to 20 carbon atoms, more preferably a linear or branched allyloxy group having 1 to 15 carbon atoms, even more preferably a linear or branched allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent a hydrogen atom, or a linear or branched chain alkyl group, alkenyl group, or aryl group having about 1 to 10 carbon atoms. Thereamong, zirconium alkoxide compounds (e.g., X⁵ is an alkoxy group) are preferable. The zirconium compounds may be used alone or in combination of two or more.

The hafnium compounds are not particularly limited so long as the amidation of the hydroxy ester compound proceeds. Specific examples of the hafnium compounds include HfX⁶₄ (wherein four X⁶s each independently represent an alkoxy group, a halogen atom, an allyloxy group, a group:-SR, a group:-NRR' or the like, and generally represent the same group). The alkoxy group of X⁶ is preferably a linear or branched alkoxy group having 1 to 10 carbon atoms, more preferably a linear or branched alkoxy group having 1 to 5 carbon atoms, even more preferably a linear or branched alkoxy group having 1 to 4 carbon atoms. The allyloxy group is preferably a linear or branched allyloxy group having 1 to 20 carbon atoms, more preferably a linear or branched allyloxy group having 1 to 15 carbon atoms, even more preferably a linear or branched allyloxy group having 1 to 10 carbon atoms. The halogen atom is preferably a chlorine atom or a bromine atom. R of the group:-SR may be a linear or branched chain alkyl group, alkenyl group, or aryl group having about 1 to 10 carbon atoms. R and R' of the group:-NRR' each independently represent a hydrogen atom, or a linear or branched chain alkyl group, alkenyl group, or aryl group having about 1 to 10 carbon atoms. Thereamong, hafnium alkoxide compounds (e.g., X⁶ is an alkoxy group) are preferable. The hafnium compounds may be used alone or in combination of two or more.

In the present invention, the catalyst may be supported by a carrier. The carrier for supporting the catalyst is not particularly limited and may be any publicly known carrier. Further, the method for supporting the catalyst on the carrier may be any publicly known method.

The amidation method of the present invention is characterized in that the ester group having a hydroxyl group at the α-, β-, γ-, or δ-position of a hydroxy ester compound is amidated with high chemoselectivity, whereas the ester group having no hydroxyl group at the α-, β-, γ-, or δ-position is unlikely to be amidated. The reason therefor is explained above using the above reaction formula (B). However, according to the conventional methods using a Schotten-Baumann reaction or peptide coupling reagents, for example, when a hydroxy ester compound further comprising an ester group different from that of an α-hydroxy ester, a β-hydroxy ester, a γ-hydroxy ester, or a δ-hydroxy ester (i.e., an ester group having no hydroxyl group at the α-, β-, γ-, or δ-position) (e.g., referring to the β-hydroxy ester compound represented by general formula (1b), at least one of group R^{b1}, group R^{c1}, group R^{d}, and group R^{e} has an ester group different from the β-hydroxy ester), the ester group having a hydroxyl group at the α-, β-, γ-, or δ-position cannot be selectively amidated. By contrast, according to the amidation method of the present invention, since the ester group having a hydroxyl group at the α-, β-, γ-, or δ-position is selectively amidated, the characteristics of the amidation of the present invention are suitably exhibited when a hydroxy ester compound further comprises an ester group different from that of an α-hydroxy ester, a β-hydroxy ester, a γ-hydroxy ester, or a δ-hydroxy ester.

Amide compounds represented by peptides have been used in a wide range of fields including pharmaceuticals, cosmetics, and functional foods. Methods for the synthesis thereof have been intensively developed as an important research subject in synthetic chemistry. However, there are few catalysts effective in the amidation which is most important in the synthesis peptides. Thus, there is no choice but to use an equivalent amount of a reagent that generates byproducts. Further, peptide synthesis, in which a multistep reaction is repeated, is very inefficient from the viewpoint of atom/economy (atomic yield). The amount of byproducts is large. There are few effective purification means. The cost of disposal of the byproducts and purification accounts for most of the necessary expense of peptide synthesis, and is one of the biggest barriers in the development in this field.

Carrying out highly stereoselective amidation is necessary for peptide synthesis using an amino acid or a derivative thereof as a raw material. Examples of the highly stereoselective amidation include enzymatic reactions *in vivo.* For example, peptides are synthesized *in vivo* with significantly high stereoselectivity by manipulating an enzyme and hydrogen bonds. However, enzyme reactions are unsuitable for mass production. If an enzyme reaction is applied to synthesis chemistry, huge financial and time costs are required.

In synthetic chemistry, amidation reactions using a catalyst have been studied. According to the conventional means, a means for activating a carboxylic acid is mainly used to form an amide bond, whereby racemization proceeds rapidly and it is difficult to synthesize a peptide with high stereoselectivity. In synthetic chemistry, a method for the synthesis of a peptide with high stereoselectivity has yet to be put into practical use. Under these circumstances, the development in highly chemoselective amidation has been desired.

As stated above, in the present invention, when a hydroxy ester compound has an amino group as a substituent (e.g., when the hydroxy ester compound has an amino acid or a derivative thereof (esterified amino acid or the like)), the amino group of an amide compound obtained by the amidation method of the present invention is used to form an amino compound, and the amino compound and the hydroxy ester compound can be subjected to an amidation reaction. In the present invention, a hydroxy ester compound having an amino group is used. Thus, the resulting amide compound can be used as an amino compound, and further, the reaction thereof with the hydroxy ester compound can be repeated. In the present invention, various structures of hydroxy ester compounds to be added by repetition can be selected to carry out the amidation reactions, whereby an amino compound comprising amino acid units having desired structures linked by peptide bonds can be synthesized and thus a desired oligopeptide (the term "oligopeptide" as used herein refers to an oligopeptide consisting of, for example, about 2 to 20, preferably about 2 to 15, even more preferably about 2 to 10 amino acid residues) can be produced with high chemoselectivity.

When at least one of the hydroxy ester compound and the amino compound is an oligopeptide consisting of, for example, 2 or more, preferably about 2 to 10 amino acid residues, the amide compound obtained by the amidation method of the present invention can be an oligopeptide. For example, a hydroxy ester compound (tripeptide) having 3 amino acid residues and an amino compound (dipeptide) having 2 amino acid residues are subjected to the amidation reaction of the present invention to synthesize an oligopeptide having 5 amino acid residues.

The amount of the catalyst used is not particularly limited, but is preferably 100 mol% or less with respect to 100 mol% of the hydroxy ester compound, preferably 20 mol% or less, more preferably about 0.1 mol% to 10 mol%.

The amidation method of the present invention can be carried out without a solvent, but is preferably carried out in an organic solvent from the viewpoint of increasing reaction efficiency. The organic solvent is not particularly limited, and examples thereof include aromatic hydrocarbons, such as toluene and xylene, alcohols such as 2,2,2-trifluoroethanol, methanol, and ethanol, ethers such as diethylether, dioxane, and tetrahydrofuran, and aprotic polar solvents such as N,N-dimethylformamide. These organic solvents may be used alone or in combination of two or more. The concentration of the hydroxy ester compound in a reaction system is not particularly limited, and is preferably 2 vol% to 70 vol% from the viewpoint of increasing reaction efficiency.

The reaction temperature of the amidation method of the present invention is not particularly limited, and is preferably about 0 °C to 150 °C from the viewpoint of increasing reaction efficiency. The reaction time is not particularly limited, and may be, for example, about 10 minutes to 72 hours.

The amidation method of the present invention can be carried out under normal pressure, under reduced pressure, or under pressure, but is preferably carried out under normal pressure from the viewpoint of facilitating the reaction.

According to the amidation method of the present invention, α-hydroxy amide compounds, β-hydroxy amide compounds, γ-hydroxy amide compounds, and δ-hydroxy amide compounds can be suitably synthesized.

Each of the hydroxy amide compounds synthesized by the amidation method of the present invention can be purified by a conventional method and can be isolated for use in various applications.

Further, in the present invention, the above catalyst comprising a compound of a transition metal of Group 4 or Group 5 of the periodic table can be suitably used in the amidation of a hydroxy ester compound, comprising reacting at least one hydroxy ester compound selected from the group consisting of an α-hydroxy ester compound, a β-hydroxy ester compound, a γ-hydroxy ester compound, and a δ-hydroxy ester compound with an amino compound to amidate an ester group having a hydroxyl group at the α-, β-, γ- or δ-position of the hydroxy ester compound.

### EXAMPLES

The following Examples and Comparative Examples are provided to more specifically describe the present invention. However, the present invention is not limited to the Examples. In the following Examples, the term "cat" refers a catalyst, and the term "rt" refers to room temperature (about 23 °C). Further, unless otherwise noted, yields were obtained using ¹H NMR with tetrachloroethane as an internal standard. Products were identified using ¹H NMR and Liquid Chromatogram Mass Spectrometry (LC-MS).

### <Example 1: Study of catalyst >

As shown in the following formula, an amidation reaction was carried out by allowing 1 molar equivalent of β-phenyl hydroxypropionate (1a) and 3 molar equivalent of para-toluidine (3a) to coexist at room temperature (about 23 °C) for 24 hours in the presence of the transition metal compound (10 mol%) listed in Table 1 in a toluene solvent. Further, 1 molar equivalent of phenyl propionate (2a) without a hydroxyl group was allowed to coexist in the reaction system to evaluate the chemo selectivity of the amidation reaction. The results are shown in Table 1.

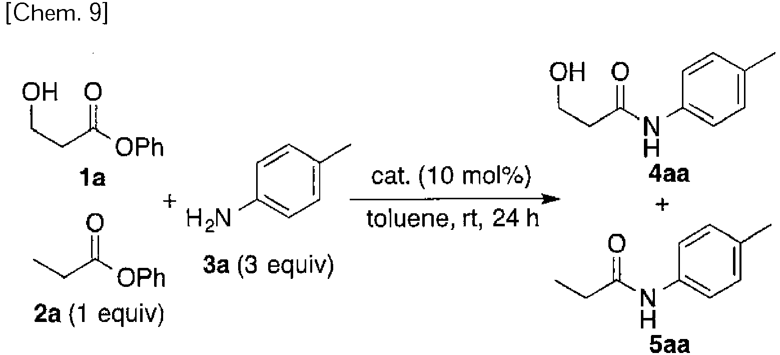

**[Table 1]**

| entry | cat. | yield of **4aa** (%)^{a} | yield of **5aa** (%)^{a} | **4aa:5aa** | entry | cat. | yield of **4aa** (%)^{a} | yield of **5aa** (%)^{a} | **4aa:5aa** |
|---|---|---|---|---|---|---|---|---|---|
| 1^{b} | La(OTf)₃ | - | - | - | 9^{b} | La(O*i*-Pr)₃ | - | - | - |
| 2 | Sc(OTf)₃ | 53 | 15 | 78:22 | 10^{b} | Ga(O*i*-Pr)₃ | - | - | - |
| 3 | In(OTf)₃ | 41 | 12 | 77:23 | 11^{b} | Yb(O*i*-Pr)₃ | - | - | - |
| 4 | Bi(OTf)₃ | 47 | 13 | 78:22 | 12 | Nb(OEt)₅ | 54 | <1 | >99:1 |
| 5 | Zr(O*t*-Bu)₄ | 11 | <1 | >99:1 | 13 | Ta(OEt)₅ | 90(85)^{c} | <1 | >99:1 |
| 6 | Ti(OEt)₅ | 31 | <1 | >99:1 | 14 | TaCl₅ | 84 | <9 | >90:10 |
| 7 | Hf(O*t*-Bu)₄ | 9 | <1 | >99:1 | 15 | TaBr₅ | 94 | <5 | >95:5 |
| 8 | VO(O*i*-Pr)₃ | 24 | <1 | >99:1 | | | | | |

La(OTf)₃ is known as a catalyst for the amidation of carboxylate esters. However, as shown in Table 1, under the present reaction conditions, the self-condensation reaction of the reaction substrate preferentially proceeded, resulting in a complicated reaction mixture (entry 1). When Sc(OTf)₃, In(OTf)₃, or Bi(OTf)₃ was used as the catalyst, though the targeted β-hydroxy amide compound was obtained in moderate yield, the amidation of phenyl propionate without a hydroxyl group proceeded at the same time (entries 2 to 4). Further, the reaction was attempted using La(o-iPr)₃, Ga(o-iPr)₃, or Yb(o-iPr)₃, resulting a complicated reaction mixture (entries 9 to 11).

In contrast, when Zr(o-tBu)₄, Ti(OEt)₅, Hf(o-tBu)₄, or VO(o-iPr)₃ was used, though the yield was not significantly high, the targeted β-hydroxy amide compound was obtained with high chemoselectivity (entries 5 to 8). Further, when Nb(OEt)₅ was used as the catalyst, the yield of the β-hydroxy amide compound was increased to 54%, and chemoselectivity was superior (entry 12). Furthermore, when the reaction was carried out in the presence of a Ta(OEt)₅ catalyst, the targeted amidation proceeded in high yield and with high chemoselectivity (entry 13). Moreover, it was revealed that when the amidation was carried out using TaCl₅ or TaBrs as a catalyst, the targeted amidation reaction was obtained in high yield and with high chemoselectivity (entries 14 and 15).

### <Example 2: Study of amino compounds>

Next, as shown in the following formula, amidation reactions were carried out by allowing 0.25 mmol of a β-hydroxy ester (1) and 0.75 mmol of various amino compounds (3) to coexist at room temperature (about 23 °C) or 100 °C for 24 hours in the presence of a Ta(OEt)₅ catalyst (10 mol%) in 1 mL of a toluene solvent. The chemical formulae and yields of the target products are shown in Table 2. The yields shown in Table 2 are isolated yields. As in Example 1, 0.25 mmol of a carboxylate ester (2) having the same structure as the β-hydroxy ester (1) except that a hydrogen atom was used in place of the hydroxyl group at the β-position coexisted in a reaction system to evaluate the chemoselectivity of the amidation reaction. In the formula, group R¹ of the β-hydroxy ester (1) is the same as described in Table 2. Further, groups R² and R³ of various amino compounds (3) each correspond to a group (different from a carbonyl group) linked to the produced amino compound listed in Table 2.

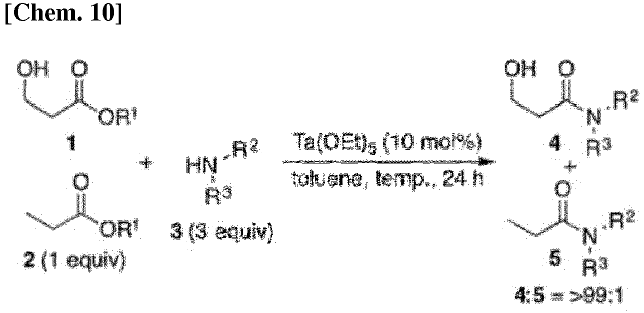

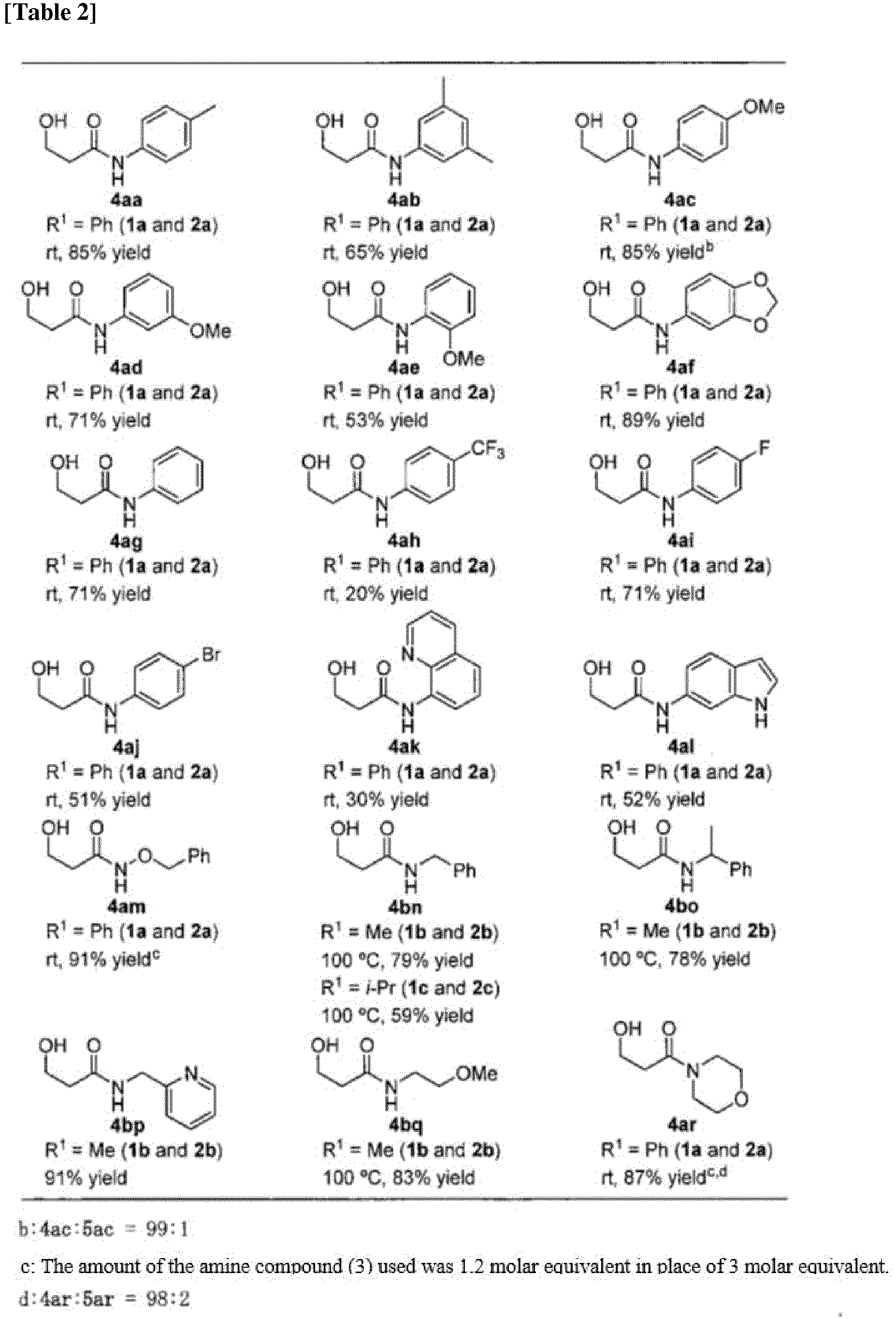

As shown in Table 2, the ratio of β-hydroxy amide compound, which was the target in the present invention, to the amide compound, which was synthesized in the case of low chemoselectivity of amidation, was (4):(5) = > 98:2 in each reaction. High chemoselectivity of the amidation of the β-hydroxy ester (1) was exhibited.

Further, aniline derivatives 3a to 3g having an electron-donating substituent exhibited particularly high reactivity in the amidation of the present invention (target products 4aa to 4ag). Aniline derivatives 3h-3i having an electron-withdrawing substituent exhibited slightly lower reactivity (target products 4ah to 4ai), but were considered to exhibit high yield and high chemoselectivity in view of conventional amidation. When aniline derivatives 3i and 3j containing a halogen atom were used, the yields of target products 4ai and 4aj were 71% and 51%, respectively. Further, it was revealed that aromatic amines 3k and 3l having a heterocycle were applicable to the present reaction (target products 4ak to 4al). β-hydroxyamide 4am obtained via a reaction with o-benzylhydroxyamine (3m) was readily converted to hydroxamic acid and a primary amide by hydrogeneration. Further, the amidation of methyl esters was possible in the present application, and β-hydroxyamides (4bn to 4bq) corresponding thereto were obtained in satisfactory yield and with high chemoselectivity. When a secondary amine morpholine (3r) was used, a target product 4ar was obtained in high yield, though the chemoselectivity thereof was slightly decreased.

### <Example 3: Study of β-hydroxy esters>

As shown in the following formula, amidation reactions were carried out by allowing 0.25 mmol of various β-hydroxy esters (1) having a substituent at the β-position and 0.75 mmol of various amino compounds (3) to coexist at room temperature (23 °C) or 100 °C for 24 hours in the presence of a Ta(OEt)₅ catalyst (10 mol%) in a toluene solvent (1 mL). The chemical formulae and yields of the target products are shown in Table 3. The yields shown in Table 3 are isolated yields. As in Example 1, 0.25 mmol of a carboxylate ester (2) having the same structure as the β-hydroxy ester (1) except that a hydroxyl group was not contained coexisted in a reaction system to evaluate the chemoselectivity of the amidation reaction. In the formula, group R¹ of the β-hydroxy ester (1) is the same as described in Table 2. Further, groups R² and R³ of the β-hydroxy ester (1) each correspond to a group linked at the β-position of the products listed in Table 3. In the formula, group R³ of the β-hydroxy ester (1) is the same as described in Table 3. Each group R⁴ of various amino compounds (3) corresponds to a group (different from a hydroxyl group and a carbonyl group) linked to the amino compound product described in Table 3.

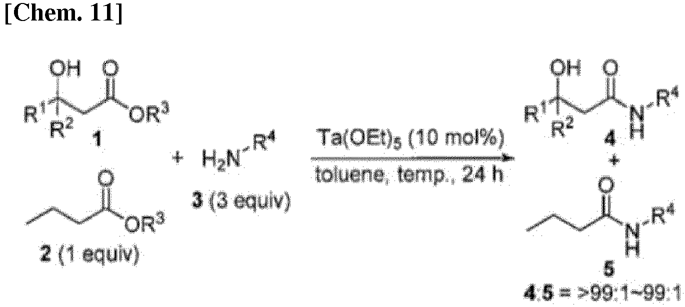

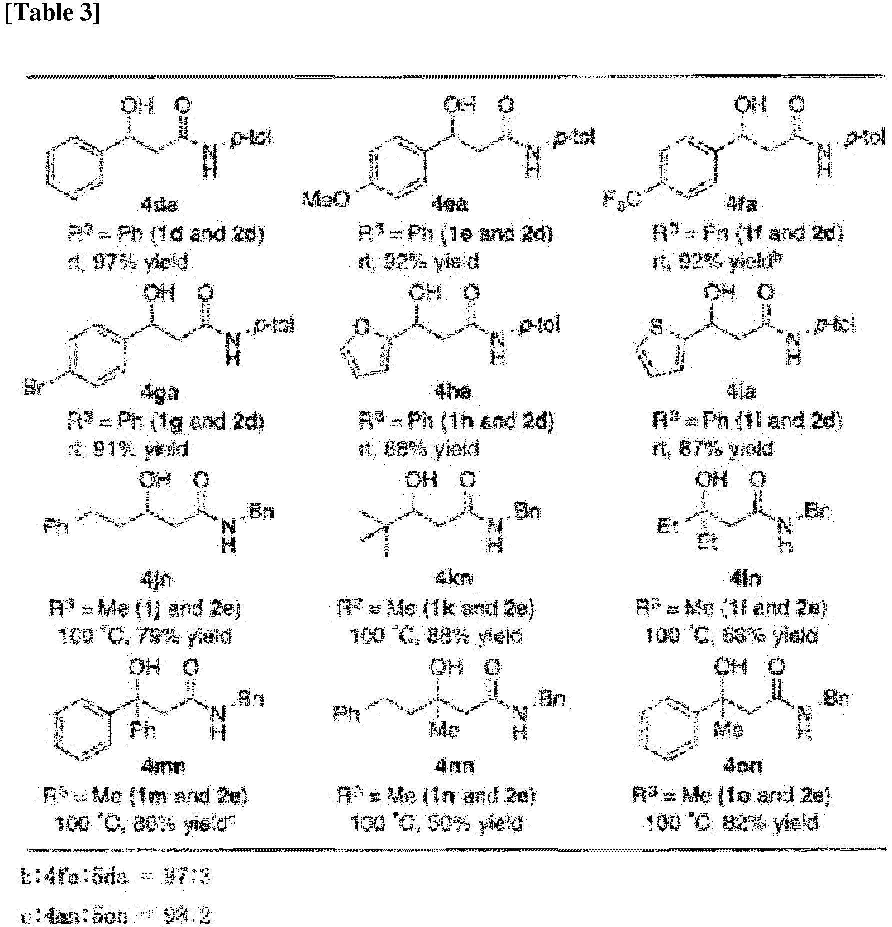

As shown in the above formula and Table 3, amidation was carried out using β-hydroxy-β-phenylpropionic acid phenyl (1d) and para-toluidine (3a) in the presence of butanoic acidphenyl without a hydroxyl group, and targeted amide compound 4da was chemoselectively obtained in a yield of 97%. Further, reaction substrates having a methoxy group, a trifluoromethyl group, or a bromine atom substituted on a benzene ring effectively reacted with para-toluidine (3a) in the presence of a tantalum catalyst, and amidated compounds 4ea to 4ga were obtained in high yield and with high chemoselectivity. Further, β-hydroxyamides 4ha and 4ia having a heterocycle were synthesized in high yield. Esters 1j and 1k having an alkyl group at the β-position reacted with benzylamine at 100 °C to provide targeted amides 4jn and 4kn in yields of 79% and 88%, respectively. When reaction substrates 11 and In substituted with two alkyl groups at the β-position were used, the yields slightly decreased. The yields of a substrate 1m substituted with two phenyl groups and a substrate 1o substituted with a phenyl group and a methyl group were higher than the yield of a reaction substrate substituted with an alkyl group (target products 4mn and 4on).

### <Example 4: Study of a molecule containing two ester groups>

As shown in the following formula, amidation reactions were carried out by allowing 0.25 mmol of compounds (6a and 6b) each having two ester groups in its molecule and 0.75 mmol of benzylamine (3n) to coexist at 100 °C or 60 °C for 24 hours in the presence of a Ta(OEt)₅ catalyst (10 mol%) in toluene solvent (1 mL). The chemical formulae and yields of the target products are shown in the following formulae.

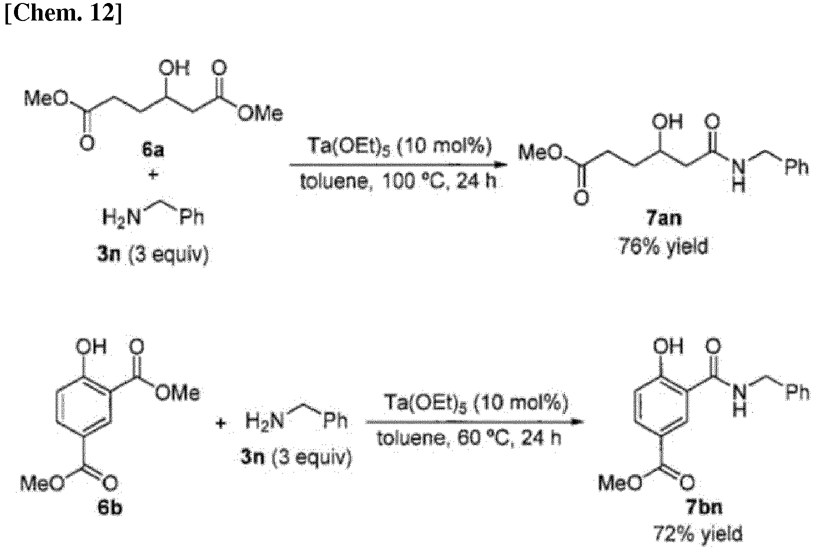

According to conventional methods using Schotten-Baumann reactions and peptide coupling reagents, it is difficult to realize chemoselective amidation reactions such as the above formulae. It was revealed that the amidation of the present invention has a very high synthetic chemical value.

### <Example 5: Synthesis of amides using various amino acid esters>

As shown in the following formula, amidation reactions were carried out by allowing 0.25 mmol of various amino acid esters (8) and 0.75 mmol of an amino compound (9) to coexist at 60 to 100 °C for 24 hours in the presence of Ta(OEt)₅ catalyst (10 mol%) in a toluene solvent (1 mL). The chemical formulae, yields, and diastereoselectivities (dr) of the resulting products are shown in Table 4. In the formulae, each group R¹ of the various amino acid ester (8) corresponds to the group linked at the β-position of the products listed in Table 4. Each group R² of the amino compound (9) corresponds to the group linked to the products listed in Table 4.

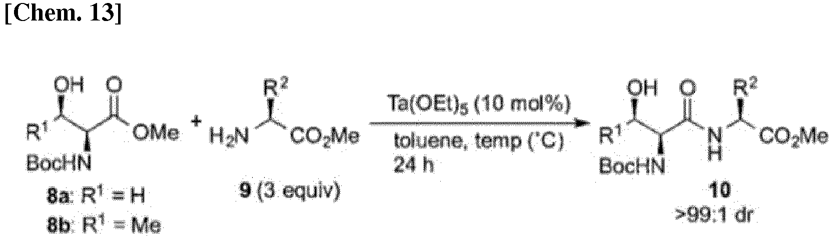

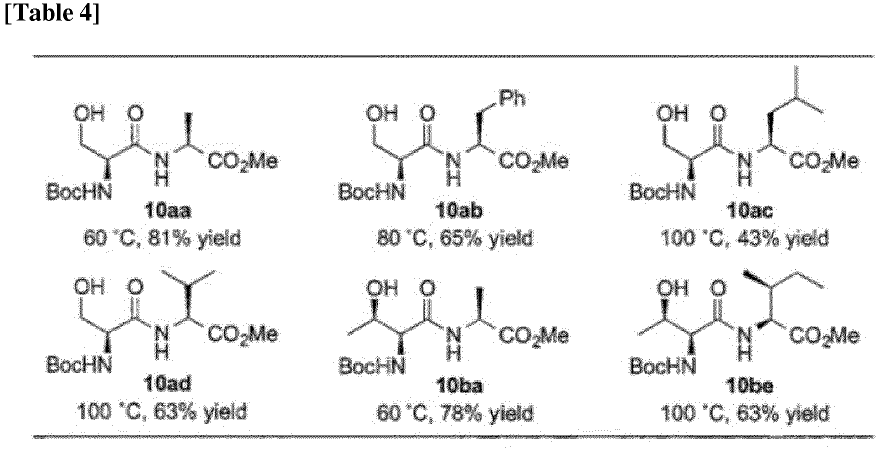

A method for the amidation of an amino acid ester using a metal catalyst to synthesize an amino acid derivative has been known. However, according to this method, it is difficult to synthesize dipeptide derivatives such as those shown in Example 5. In contrast, in, Example 5, the conversion of amino acid esters having a hydroxyl group, such as a serinemethyl ester and a threoninemethyl ester, in the presence of a tantalum catalyst into dipeptides was attempted. The targeted dipeptides 10aa to lObe were diastereoselectively obtained in satisfactory yield.

### <Example 6: Study of various hydroxy esters>

As shown in the following formulae (6-1) to (6-7), amidation reactions were carried out by allowing (1 equivalent of) various hydroxy esters having a hydroxyl group at the α-, β-, γ-, or δ-position of the ester group and (3.0 equivalent of) various amino compounds to coexist at room temperature (23 °C) or at 100 °C in the presence of Ta(OEt)₅ catalyst (10 mol%) in a toluene solvent (1 mL). Further, as shown in the following formulae, as in Example 1, reactions were carried out by allowing an ester compound without a hydroxyl group to coexist in a reaction system to evaluate the chemoselectivity of the amidation reactions (formulae (6-1), (6-2), (6-4), and (6-5)). Furthermore, a reaction was carried out by allowing a β-hydroxy ester compound and a δ-hydroxy ester compound to coexist (formula (6-6)). Formula (6-7) collectively shows reactions of hydroxy esters wherein n is 0, 3, or 4. The chemical formula and the yield of a product are shown in each formula.

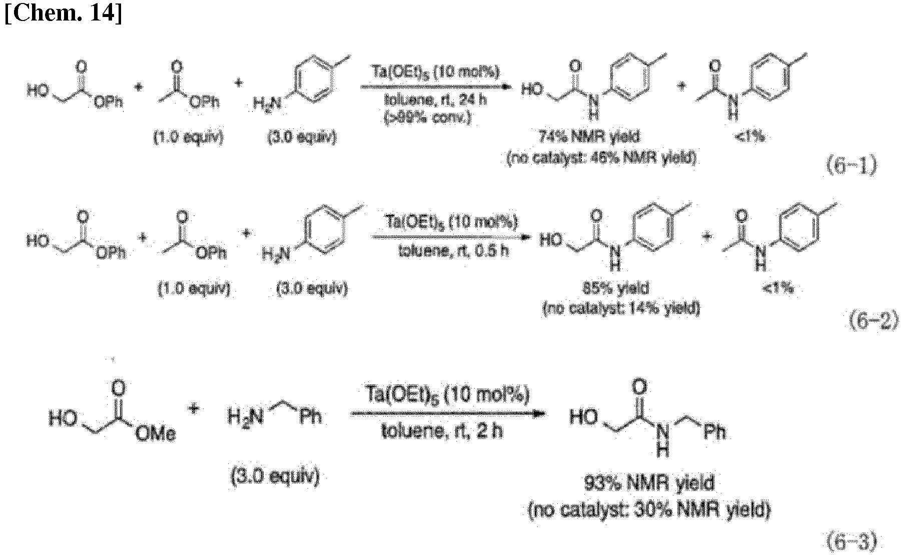

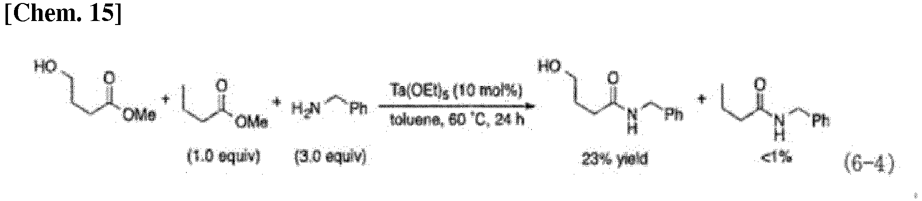

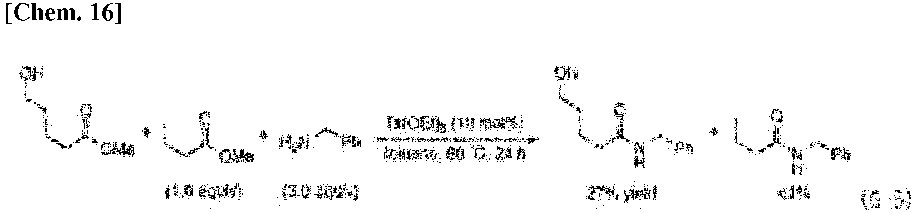

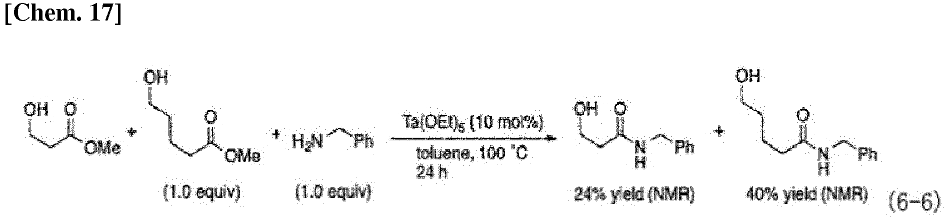

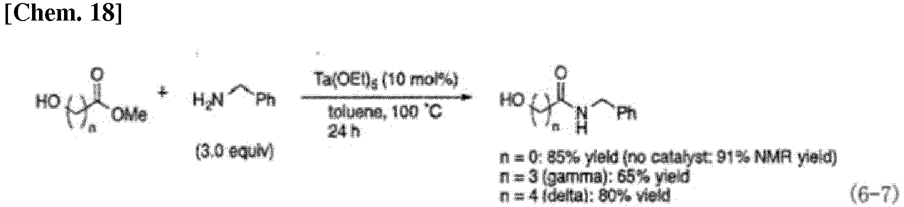

The results of formulae (6-1) to (6-7) in Example 6 revealed that according to the amidation method of the present invention, all of the α-hydroxy ester compound, β-hydroxy ester compound, γ-hydroxy ester compound, and δ-hydroxy ester compound were highly chemo selectively amidated.

### <Example 7: Synthesis of a tripeptide>

As shown in the following formula, 1.0 equivalent of L-serinemethyl ester (Cbz-L-Ser-OMe) (i.e., a β-hydroxy ester) having an amino group protected by a benzyloxycarbonyl group (Cbz) and 1.1 equivalent of t-butyl ester (i.e., an amino compound) of a dipeptide of L-serine and L-alanine were allowed to coexist at room temperature (23 °C) in the presence of a Ta(OEt)₅ catalyst (10 mol%) in a toluene solvent (1 mL), and an amidation reaction was carried out at 100 °C for 18 hours. As a result, the tripeptide shown in the following formula was obtained (yield 50%).

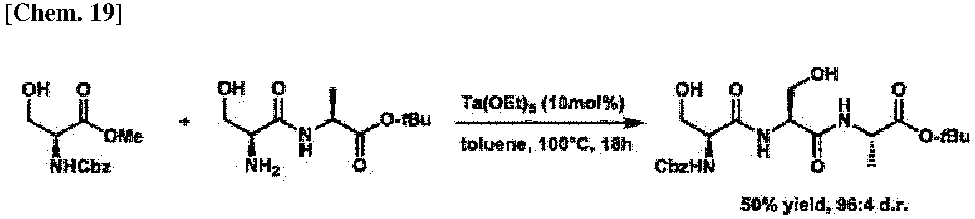

### <Example 8: Synthesis of a tripeptide>

As shown in the following formula, 1.0 equivalent of serinepropargylester (Cbz-L-Ser-OCH₂CCH) (i.e., a β-hydroxy ester) having an amino group protected by a benzyloxycarbonyl group (Cbz) and 1.1 equivalent of t-butyl ester (i.e., an amino compound) of a dipeptide of L-serine and L-alanine were allowed to coexist at room temperature (23 °C) in the presence of a Ta(OEt)₅ catalyst (10 mol%) in a toluene solvent (1 mL), and an amidation reaction was carried out at 60 °C for 42 hours. As a result, the tripeptide shown in the following formula was obtained (yield 72%).

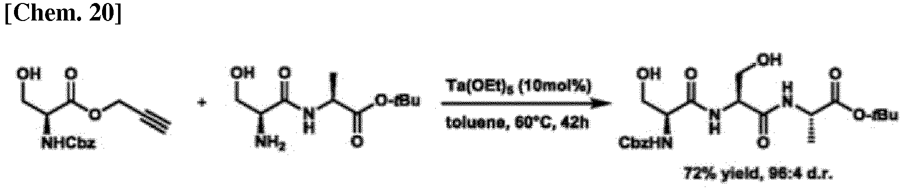

### <Example 9: Synthesis of an oligopeptide (pentamer)>

As shown in the following formula, 1.0 equivalent of, as a β-hydroxy ester, a tripeptide methyl ester 11 (i.e., a β-hydroxy ester) having an amino group protected by a benzyloxycarbonyl group (Cbz) and 2 or 3 equivalent of, as an amino compound, a t-butyl ester 12 (i.e., an amino compound) of a dipeptide of L-serine and L-alanine were allowed to coexist at room temperature (23 °C) in the presence of a Ta(OEt)₅ catalyst (10 mol%) in a solvent (1 to 3 mL), and amidation reactions were carried out at 60 °C for 24 hours. As a result, the tripeptide 13 (an amino acid pentamer derivative) shown in the following formula was obtained. The yields thereof are shown in Table 5.

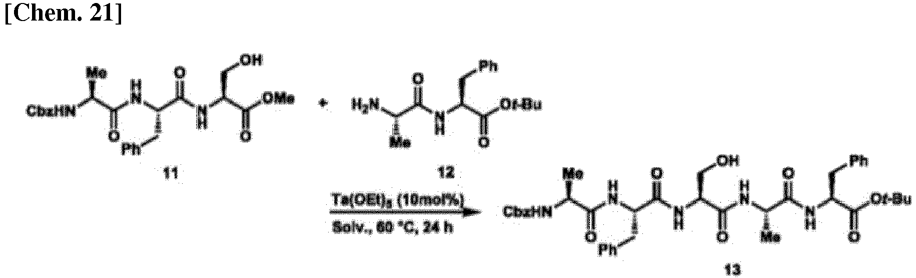

**[Table 5]**

| Entry | solv.(mL) | **12** (eq.) | yield of **13** (%) |
|---|---|---|---|
| 1 | toluene (1) | 3 | 26 |
| 2 | EtOH (1) | 3 | 34 |
| 3 | CF₃CH₂OH (1) | 3 | 13 |
| 4 | THF (3) | 2 | 22 |
| 5 | CF₃CH₂OH (3) | 2 | 17 |
| 6 | MeOH (3) | 2 | 37 |
| 7 | DMF (3) | 2 | 33 |

The yields in Table 5 were measured as conversion rates by HPLC analysis.

### <Example 10: Synthesis of an oligopeptide (heptamer)>

As shown in the following formula, 1.0 equivalent of the β-hydroxy ester 14 (AcHN-Ala-Phe-Val-Ala-Thr-COOMe) shown in the following formula and 2.0 equivalent of the amino compound 15 (H₂N-Ala-Phe-COOtBu) were allowed to coexist at room temperature (23 °C) in the presence of catalysts (100 mol%) in a methanol solvent (0.3 mL), and amidation reactions were carried out at 60 °C for 24 hours. As a result, the tripeptide 16 (an amino acid heptamer derivative) shown in the following formula was obtained. The yields thereof are shown in Table 6.

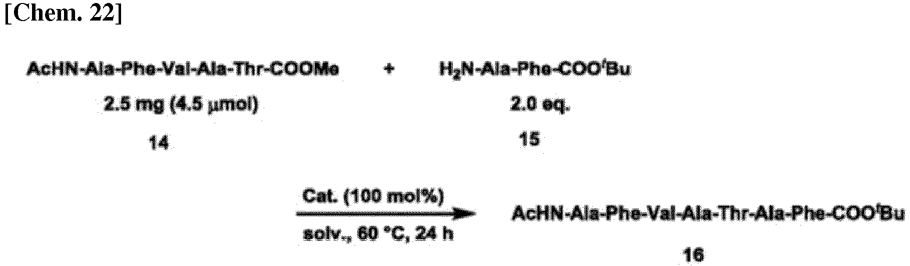

**[Table 6]**

| Entry | solv.(mL) | cat. | yield of **16** (%) |
|---|---|---|---|
| 1 | MeOH (0.3) | Ta(OEt)₅ | 36.5 |
| 2 | MeOH (0.3) | Nb(OEt)₅ | 52.1 |

The yields were measured as conversion rates by HPLC analysis.

### <Example 11: Synthesis of an oligopeptide (octamer)>

As shown in the following formula, 1.0 equivalent of the β-hydroxy ester 14 (AcHN-Ala-Phe-Val-Ala-Thr-COOMe) shown in the following formula and 2.0 equivalent of the amino compound 17 (H₂N-Ala-Phe-Ile-COOBn) were allowed to coexist at room temperature (23 °C) in the presence of catalysts (100 mol%) without a solvent or in solvents (0.3mL), and amidation reactions were carried out at 60 °C for 24 hours. As a result, the tripeptide 18 (amino acid octamer derivative) shown in the following formula was obtained. The yields thereof are shown in Table 7.

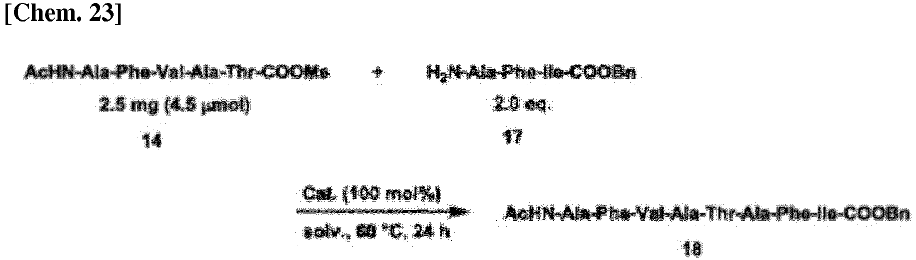

**[Table 7]**

| Entry | solv.(mL) | cat. | yield of **18** (%) |
|---|---|---|---|
| 1 | none | Ta(OEt)₅ | 21.2 |
| 2 | none | Nb(OEt)₅ | 35.0 |
| 3 | MeOH (0.3) | Ta(OEt)₅ | 7.2 |
| 4 | EtOH (0.3) | Ta(OEt)₅ | 6.2 |
| 5 | DMF (0.3) | Ta(OEt)₅ | 21.8 |

The yields in Table 7 were measured as conversion rates by HPLC analysis.

## Claims

1. A method for the amidation of a hydroxy ester compound, comprising reacting at least one hydroxy ester compound selected from the group consisting of α-hydroxy ester compounds, β-hydroxy ester compounds, γ-hydroxy ester compounds, and δ-hydroxy ester compounds with an amino compound in the presence of a catalyst comprising a compound of a transition metal of Group 4 or Group 5 of the periodic table to amidate an ester group having a hydroxyl group at the α-, β-, γ- or δ-position of the hydroxy ester compound.

2. The method for the amidation of a hydroxy ester compound according claim 1, wherein the hydroxy ester compound is represented by any of the following formulae (1a) to (1d): 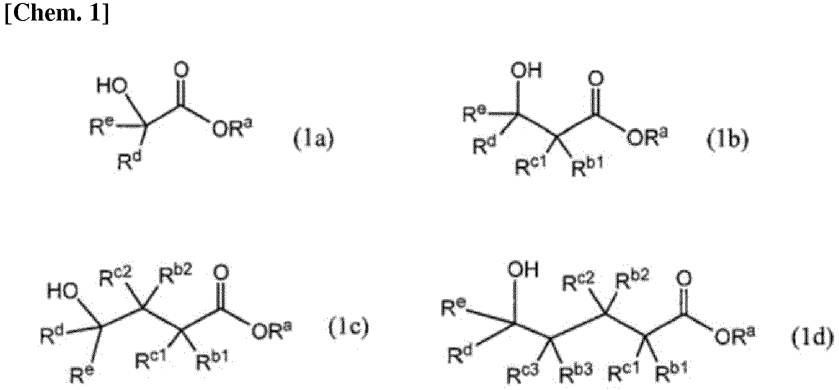 wherein group R^{a} represents an optionally substituted aliphatic group, an optionally substituted aromatic group, an optionally substituted alicyclic group, or an optionally substituted heterocyclic group; group R^{b1}, group R^{b2}, group R^{b3}, group R^{c1}, group R^{c2}, and group R^{c3} each independently represent a hydrogen atom, a halogen atom, a hydroxy group, an optionally substituted aliphatic group, an optionally substituted aromatic group, an optionally substituted alicyclic group, or an optionally substituted heterocyclic group; group R^{d} and group R^{e} each independently represent a hydrogen atom, an optionally substituted aliphatic group, an optionally substituted aromatic group, an optionally substituted alicyclic group, or an optionally substituted heterocyclic group; and group R^{b1} and group R^{c1}, group R^{b1} and group R^{b2}, group R^{b1} and group R^{b3}, group R^{b2} and group R^{c2}, group R^{b2} and group R^{b3}, group R^{b3} and group R^{c3}, group R^{b1} and group R^{d}, group R^{b2} and group R^{d}, or group R^{b3} and group R^{d} may be linked together to form a ring structure.

3. The method for the amidation of a hydroxy ester compound according claim 1 or 2, wherein the amino compound is represented by the following formula (3): 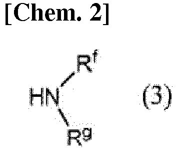 wherein group R^{f} and group R^{g} each independently represent a hydrogen atom, an optionally substituted aliphatic group, an optionally substituted aromatic group, an optionally substituted alicyclic group, an optionally substituted heterocyclic group, or group R^{f} and group R^{g} may form a saturated or unsaturated heterocycle together with a linked nitrogen atom, provided that the heterocycle may have a substituent.

4. The method for the amidation of a hydroxy ester compound according any one of claims 1 to 3, wherein the compound of a transition metal is at least one selected from the group consisting of titanium compounds, zirconium compounds, hafnium compounds, vanadium compounds, niobium compounds, and tantalum compounds.

5. The method for the amidation of a hydroxy ester compound according any one of claims 1 to 4, wherein the amount of the catalyst used is 100 mol% or less per 100 mol% of the hydroxy ester compound.

6. The method for the amidation of a hydroxy ester compound according any one of claims 1 to 5, wherein amidation is performed in an organic solvent at a reaction temperature of 0 to 150 °C for a reaction time of 10 minutes to 72 hours.

7. The method for the amidation of a hydroxy ester compound according any one of claims 1 to 6, wherein the hydroxy ester compound further comprises an ester group other than an α-hydroxy ester, a β-hydroxy ester, a γ-hydroxy ester, or a δ-hydroxy ester.

8. The method for the amidation of a hydroxy ester compound according any one of claims 1 to 7, wherein the amino compound is an amino acid, a salt thereof, an amino-acid ester, or a salt thereof.

9. The method for the amidation of a hydroxy ester compound according any one of claims 1 to 8, wherein the hydroxy ester compound further comprises an amino group.

10. The method for the amidation of a hydroxy ester compound according any one of claims 1 to 9, wherein at least either one of the hydroxy ester compound and the amino compound is an oligopeptide comprising 2 or more amino acid residues.

11. The method for the amidation of a hydroxy ester compound according any one of claims 1 to 10, wherein an amide compound obtained by amidation is at least one selected from the group consisting of the following general formula (4a) to (4d) 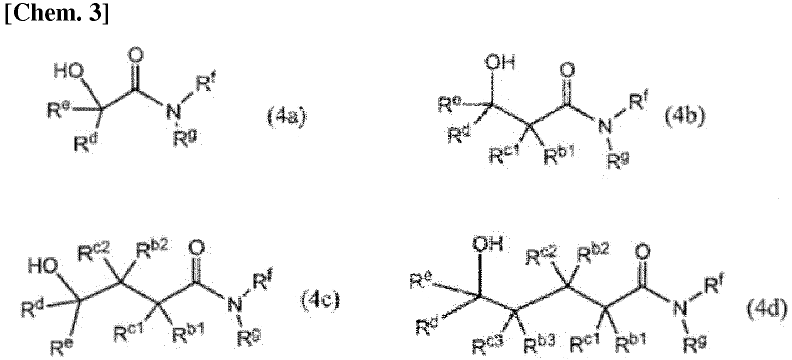 wherein group R^{a} represents an optionally substituted aliphatic group, an optionally substituted aromatic group, an optionally substituted alicyclic group, or an optionally substituted heterocyclic group; group R^{b1}, group R^{b2}, group R^{b3}, group R^{c1}, group R^{c2}, and group R^{c3} each independently represent a hydrogen atom, a halogen atom, a hydroxy group, an optionally substituted aliphatic group, an optionally substituted aromatic group, an optionally substituted alicyclic group, or an optionally substituted heterocyclic group; group R^{d} and group R^{e} each independently represent a hydrogen atom, an optionally substituted aliphatic group, an optionally substituted aromatic group, an optionally substituted alicyclic group, or an optionally substituted heterocyclic group; group R^{b1} and group R^{c1}, group R^{b1} and group R^{b2}, group R^{b1} and group R^{b3}, group R^{b2} and group R^{c2}, group R^{b2}; and group R^{b3}, group R^{b3} and group R^{c3}, group R^{b1} and group R^{d}, group R^{b2} and group R^{d}, or group R^{b3} and group R^{d} may be linked together to form a ring structure; and group R^{f} and group R^{g} each independently represent a hydrogen atom, an optionally substituted aliphatic group, an optionally substituted aromatic group, an optionally substituted alicyclic group, an optionally substituted heterocyclic group, or group R^{f} and group R^{g} may form a saturated or unsaturated heterocycle together with a linked nitrogen atom, provided that the heterocycle may have a substituent.

12. A catalyst comprising a compound of a transition metal of Group 4 or Group 5 of the periodic table, the catalyst being used in a method for the amidation of a hydroxy ester compound, and the method comprising reacting at least one hydroxy ester compound selected from the group consisting of an α-hydroxy ester compound, a β-hydroxy ester compound, a γ-hydroxy ester compound, and a δ-hydroxy ester compound with an amino compound to amidate an ester group having a hydroxyl group at the α-, β-, γ- or δ-position of the hydroxy ester compound.
